# EUROPEAN PATENT APPLICATION

(11) **EP 4 342 411 A1**
(43) Date of publication of application: **27.03.2024**
(21) Application number: 21940797.0
(22) Date of filing: 20.05.2021
(51) Int. Cl.: A61B 34/20

(54) **MEDICAL SYSTEM AND NAVIGATION METHOD**

(71) Applicant: Asahi Intecc Co., Ltd., Seto-shi, Aichi 489-0071 (JP)
(72) Inventor: OSHIMA, Fumiyoshi, Seto-shi, Aichi 489-0071 (JP)
(74) Representative: Winter, Brandl - Partnerschaft mbB
(86) International application number: PCT/JP2021/019158
(87) International publication number: WO 2022/244183

(57) **Abstract**

A medical system includes a distal end information acquisition unit that acquires distal end rotation information regarding a rotation movement of a distal end part of a medical device to be inserted into a living body and a proximal end information acquisition unit that acquires proximal end rotation information regarding a rotation movement of a proximal end part of the medical device to be operated by an operator.

## Description

### TECHNICAL FIELD

The present inventions relate to a medical system and a navigation method.

### BACKGROUND ART

With respect to a medical device such as a guide wire or a catheter, technologies for grasping a position of the medical device in a living body are known. For example, Patent Literature 1 discloses a technology in which a magnetization region is provided in a distal end part of a medical device, and a position of the distal end part of the medical device is obtained by using a measurement value from a detector head disposed outside a body. For example, Patent Literature 2 discloses a technology in which an electrode is provided in the distal end part of a catheter, and a position of the distal end part of the catheter is obtained by using a current distribution generated by causing a current to flow between the electrode and an electrode disposed outside a body. In addition, Patent Literature 3 discloses a technology of acquiring rotation of a proximal end part of a medical device by fixing a gyroscope sensor for measuring an angular position and a rotation angle of the proximal end part to the proximal end part of the medical device.

### CITATION LIST

### Patent Literature

Patent Literature 1: JP 2019-520129 W
Patent Literature 2: JP 2012-125579 A
Patent Literature 3: JP 2019-171074 A

### SUMMARY OF INVENTION

### TECHNICAL PROBLEM

In a technique such as percutaneous coronary intervention (PCI), an operator needs to cause a medical device to reach a specific blood vessel (hereinafter, also referred to as a "target blood vessel") having a lesion such as stenosis or occlusion in an intricate network of blood vessels. Therefore, the operator adds a small curved shape or the like to a distal end part of the medical device, and selects a desired blood vessel leading to the target blood vessel while rotating the distal end part of the medical device. However, it is known that rotation at a proximal end part is not directly transmitted to the distal end part, in other words, loss torque is generated, because the medical device has a very small outer diameter while having an elongated outer shape with a length of about several meters.

Conventionally, since the operator operates the medical device by feeling of hand and experience while considering such loss torque, there is a problem in that it takes time and effort for the technique and high skill is required for the operator. In this regard, in the technologies described in Patent Literatures 1 and 2, no consideration is given to the rotation of the distal end part and the proximal end part of the medical device. Further, in the technology described in Patent Literature 3, no consideration is given to the rotation of the distal end part of the medical device, and no consideration is given to the above-described problem caused by the loss torque of the medical device. Note that such a problem is generally common across medical devices such as a catheter, and a guide wire which are inserted into an organ (biological tube) in the human body such as a blood vessel system, a lymphatic gland system, a biliary tract system, a urinary tract system, a respiratory tract system, a digestive organ system, a secretory gland, or a reproductive organ.

The present inventions have been made to solve at least a part of the above-described problems, and an object of the present inventions is to provide a medical system capable of acquiring rotation movements of a proximal end part and a rotation movement of a distal end part of a medical device.

### SOLUTION TO PROBLEM

The present inventions have been made to solve at least a part of the above-described problems, and can be implemented as the following aspects.
(1) According to an aspect of the disclosed embodiments, a medical system is provided. The medical system includes a distal end information acquisition unit that acquires distal end rotation information regarding a rotation movement of a distal end part of a medical device to be inserted into a living body and a proximal end information acquisition unit that acquires proximal end rotation information regarding a rotation movement of a proximal end part of the medical device to be operated by an operator.
   According to this configuration, the distal end information acquisition unit can acquire quantitative distal end rotation information regarding the rotation movement of the distal end part of the medical device. Similarly, the proximal end information acquisition unit can acquire quantitative proximal end rotation information regarding the rotation movement of the proximal end part of the medical device. That is, according to this configuration, it is possible to provide a medical system capable of acquiring the rotation movement of the proximal end part and the rotation movement of the distal end part of the medical device. For this reason, it is possible to contribute to improvement in accuracy and improvement in efficiency of a technique by using the acquired rotation movements of the proximal end part and the distal end part in various methods. For example, the acquired rotation movements of the proximal end part and the distal end part may be directly presented to the operator, or may be used to derive the rotation movement of the distal end part following the rotation movement of the proximal end part.
(2) The medical system of the aspect described above may further include a correspondence relation deriving unit that derives, by using the proximal end rotation information and the distal end rotation information, a correspondence relation between the rotation movement of the proximal end part of the medical device and the rotation movement of the distal end part of the medical device following the rotation movement of the proximal end part, and a request operation output unit that outputs, by using the correspondence relation derived by the correspondence relation deriving unit, a request operation of the proximal end part of the medical device, which is estimated with respect to a target operation of the distal end part of the medical device.
   According to this configuration, the correspondence relation deriving unit can derive, by using the quantitative proximal end rotation information and the distal end rotation information, the correspondence relation between the rotation movement of the proximal end part of the medical device and the rotation movement of the distal end part of the medical device following the rotation movement of the proximal end part. In addition, the request operation output unit can estimate and output the "request operation of the proximal end part of the medical device" in consideration of loss torque of the medical device by using the correspondence relation derived by the correspondence relation deriving unit. Here, the "request operation of the proximal end part of the medical device" is a request operation of the proximal end part that is estimated with respect to the target operation of the distal end part of the medical device, and in other words, means an estimation of what kind of operation the operator performs on the proximal end part of the medical device when attempting to perform an arbitrary target operation with respect to the distal end part of the medical device. As a result, according to the present configuration, it is possible to provide a medical system capable of outputting the request operation of the proximal end part of the medical device, which is estimated with respect to the target operation of the distal end part of the medical device, and thus it is possible to improve the accuracy of the technique and the efficiency of the technique.
(3) In the medical system according to the aspects described above, the request operation output unit may output, as the request operation, an operation of the proximal end part of the medical device, which is estimated with respect to the target operation of the distal end part necessary for moving a position of the distal end part in a cross sectional direction in a blood vessel from a current position to a target position by the rotation movement of the distal end part of the medical device, the medical system may further include a target position acquisition unit that acquires position information of the target position, the distal end information acquisition unit may further acquire distal end position information representing a current position of the distal end part, and the request operation output unit may output the request operation by using the position information of the target position and the distal end position information.
   According to this configuration, the medical system further includes the target position acquisition unit that acquires the position information of the target position of the distal end part of the medical device, and the distal end information acquisition unit acquires the distal end position information representing the position of the distal end part of the medical device. Therefore, the request operation output unit can specify the target position of the distal end part of the medical device in the cross sectional direction in the blood vessel by the position information of the target position. Similarly, the request operation output unit can specify the current position of the distal end part of the medical device in the cross sectional direction in the blood vessel by the distal end position information. As a result, the request operation output unit can specify the target operation of the distal end part necessary for moving the position of the distal end part of the medical device from the current position to the target position, and can output the request operation necessary for the target operation.
(4) In the medical system according to the aspects described above, the request operation output unit may output, as the request operation, a rotation direction and a rotation angle of the proximal end part of the medical device.
   According to this configuration, since the request operation output unit outputs the rotation direction and the rotation angle of the proximal end part of the medical device as the request operation, it is possible to clearly present a request operation content to the operator.
(5) In the medical system according to the aspects described above, the correspondence relation deriving unit may further derive the correspondence relation by using information regarding a shape of the blood vessel and information regarding a physical property of the medical device.
   According to this configuration, since the correspondence relation deriving unit further derives the correspondence relation by using the information regarding the shape of the blood vessel and the information regarding the physical property of the medical device, it is possible to improve the accuracy of the obtained correspondence relation. As a result, it is possible to improve estimation accuracy of the request operation to the target operation in the request operation output unit.
(6) The medical system according to the aspects described above may further include the medical device with a holder attached to the proximal end, the holder may have a hollow main body part that has an insertion path for the medical device, a gripping part that is provided in the insertion path and grips the proximal end part of the medical device, and a detector that detects information regarding the rotation movement of the proximal end part of the medical device gripped by the gripping part, the proximal end information acquisition unit may acquire information detected by the detector as the proximal end rotation information.
   According to this configuration, the holder is configured as a so-called torquer including the hollow main body part, the gripping part that grips the proximal end part of the medical device, and the detector. In addition, since the proximal end information acquisition unit acquires information detected by the detector of the holder (torquer) as the proximal end rotation information, it is possible to easily construct a function related to the proximal end information acquisition unit in the medical system.
(7) In the medical system according to the aspects described above, the holder may further have a motor that rotates the proximal end part of the medical device gripped by the gripping part; and a control unit that controls driving of the motor, the request operation output unit may transmit the request operation to the control unit, and the control unit may control the driving of the motor in accordance with the request operation received.
   According to this configuration, the holder further has the motor that rotates the proximal end part of the gripped medical device and the control unit, and the control unit controls the driving of the motor in accordance with the request operation received from the request operation output unit. Therefore, it is possible to provide a medical system capable of automatically operating the proximal end part of the medical device in accordance with the request operation.
(8) The medical system according to the aspects described above may further include a magnetic sensor, the medical device may have a first magnetization part that is provided at the distal end part and magnetized, and a second magnetization part that is provided closer to a proximal end side than the first magnetization part and magnetized, the magnetic sensor may detect magnetic fields generated from the first magnetization part and the second magnetization part, and the distal end information acquisition unit may calculate the distal end rotation information by using the magnetic fields detected by the magnetic sensor.
   According to this configuration, the medical device has the first magnetization part and the second magnetization part which are magnetized, and the medical system further includes the magnetic sensor which detects the magnetic fields generated from the first magnetization part and the second magnetization part. In addition, since the distal end information acquisition unit calculates the distal end rotation information by using the magnetic fields detected by the magnetic sensor, it is possible to easily construct a function related to the distal end information acquisition unit in the medical system.

Note that the present inventions can be achieved in various modes, and can be achieved in modes such as an information processing apparatus, a holder, a medical device, an examination apparatus, a system including these apparatuses, a computer program for achieving functions of these apparatuses and system, a server apparatus for distributing the computer program, a non-transitory storage medium storing the computer program, and the like.

### BRIEF DESCRIPTION OF DRAWINGS

Fig. 1 is an explanatory diagram exemplifying a configuration of a medical system.
Fig. 2 is an explanatory diagram exemplifying a configuration of a guide wire.
Fig. 3 is an explanatory diagram exemplifying a configuration of a holder.
Fig. 4 is an explanatory diagram exemplifying a cross sectional configuration along a line A-A in Fig. 3.
Fig. 5 is a flowchart illustrating an example of a navigation process.
Fig. 6 is an explanatory diagram illustrating a state of a distal end part of the guide wire in a blood vessel.
Fig. 7 is a diagram for explaining steps S22 to S28 in Fig. 5.
Figs. 8A and 8B illustrate another example of an image output to a display screen.
Fig. 9 is an explanatory diagram exemplifying a configuration of a medical system of a second embodiment.
Fig. 10 is a flowchart illustrating an example of a navigation process of the second embodiment.
Fig. 11 is an explanatory diagram exemplifying a configuration of a medical system of a third embodiment.
Fig. 12 is an explanatory diagram exemplifying a configuration of a holder of the third embodiment.
Fig. 13 is a flowchart illustrating an example of a navigation process of the third embodiment.
Fig. 14 is an explanatory diagram exemplifying a configuration of a medical system of a fourth embodiment.
Fig. 15 is an explanatory diagram exemplifying a configuration of a medical system of a fifth embodiment.
Fig. 16 is a flowchart illustrating an example of a navigation process of the fifth embodiment.
Fig. 17 is an explanatory diagram exemplifying a configuration of a medical system of a sixth embodiment.
Fig. 18 is a flowchart illustrating an example of a navigation process of the sixth embodiment.

### EMBODIMENTS OF THE INVENTION

### <First Embodiment>

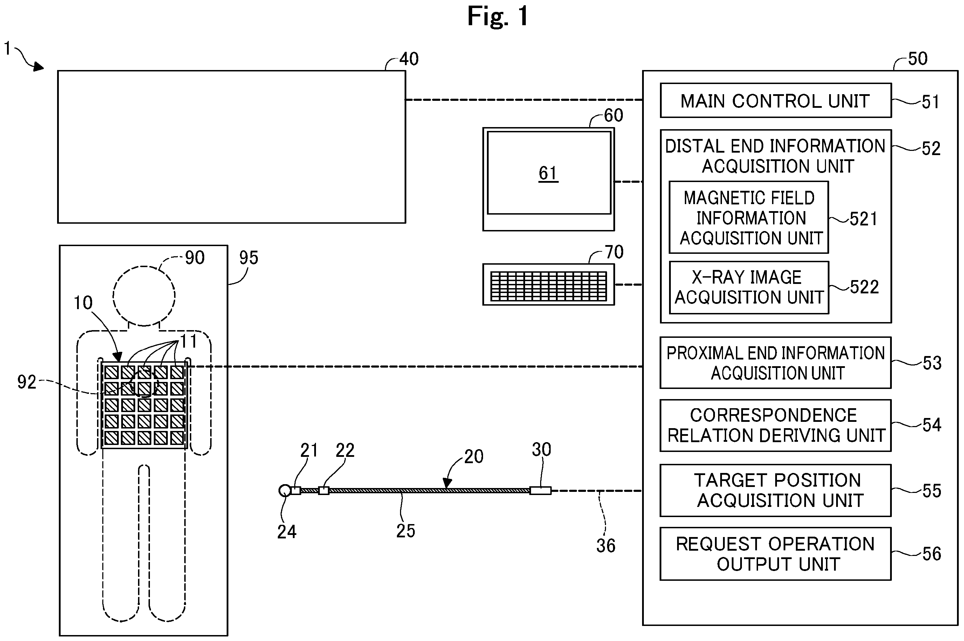

Fig. 1 is an explanatory diagram exemplifying a configuration of a medical system 1. The medical system 1 is an apparatus used for treatment or diagnosis of a living body (here, a human body) 90, and can output a request operation of a proximal end part of a medical device, which is estimated with respect to a target operation of a distal end part of the medical device. Here, the "target operation" is an arbitrary operation which is set with respect to the distal end part of the medical device, and for example, "an operation for positioning the distal end part of the medical device at a center of a blood vessel in a cross sectional direction of the blood vessel" or the like can be exemplified. In addition, the "request operation" indicates what kind of operation the operator performs on the proximal end part of the medical device when attempting to perform the target operation described above, and for example, "an operation of rotating the proximal end part of the medical device to a left side by 90 degrees" or the like can be exemplified.

The medical system 1 includes a magnetic sensor array 10, a guide wire 20 as a medical device, a holder 30 attached to a proximal end part of the guide wire 20, an X-ray imaging apparatus 40, a computer 50, a monitor 60, and an operation unit 70.

The magnetic sensor array 10 is an apparatus that detects strengths, orientations, and the like of magnetic fields generated by a human body 90 and the guide wire 20. A plurality of magnetic sensors 11 are arranged on the magnetic sensor array 10, and the magnetic sensors 11 are arranged vertically and horizontally in a matrix form. The magnetic sensor 11 is a detection element that detects biomagnetic field information, and examples thereof may include a GHz-Spin-Rotation (GSR) sensor, a magnetoresistive effect element (MR), a Magneto Impedance element (MI), and a superconducting quantum interference device (SUQUID).

The magnetic sensor array 10 is located near a center portion of a bed 95 on which the human body 90 lies. The magnetic sensor array 10 may be configured to be attached to the human body 90 during treatment or diagnosis. Furthermore, the magnetic sensor array 10 may be configured to be attached to the human body 90 during treatment. For example, the magnetic sensor array 10 may be configured to have a band shape to be wrapped around the human body 90 or may be configured to have a garment or cap shape. In these cases, the magnetic sensors 11 may be arranged along a shape of the human body 90. In addition, the magnetic sensor array 10 may be composed of two or more plates which are three-dimensionally arranged on one or both of the front and back of the human body, and on one or both of the sides of the human body.

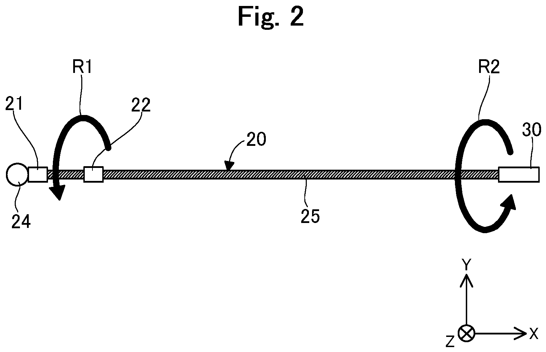

Fig. 2 is an explanatory diagram exemplifying a cross sectional configuration of the guide wire 20. Fig. 2 illustrates X-, Y-, and Z-axes that are orthogonal to one another. The X-axis corresponds to a longitudinal direction of the guide wire 20, the Y-axis corresponds to a height direction of the guide wire 20, and the Z-axis corresponds to a width direction of the guide wire 20. The left side (-X-axis direction) in Fig. 2 is referred to as a "distal end side" of the guide wire 20 and each component member, and the right side (+X-axis direction) in Fig. 2 is referred to as a "proximal end side" of the guide wire 20 and each component member. In addition, one end positioned on the distal end side of the both ends in the longitudinal direction (X-axis direction) of the guide wire 20 and each component member is referred to as a "distal end," and the other end positioned on the proximal end side is referred to as a "proximal end." Furthermore, the distal end and a vicinity thereof are referred to as a "distal end part," and the proximal end and a vicinity thereof are referred to as a "proximal end part." The distal end side is inserted into a living body, and the proximal end side is operated by an operator such as a surgeon.

The guide wire 20 is a device that is inserted into a blood vessel of the human body 90 and advances in the blood vessel ahead of a catheter or the like. The guide wire 20 includes a main body part 25, a first magnetization part 21, a second magnetization part 22, and a distal tip 24. The main body part 25 has an elongated outer shape and is a member formed of any resin material or any metal material. The main body part 25 may be composed only of a core shaft, or may be composed of a core shaft and a coil body.

The first magnetization part 21 is provided, in the distal end part of the main body part 25, closer to the proximal end side than the distal tip 24 and closer to the distal end side than the second magnetization part 22. The first magnetization part 21 is a substantially cylindrical or substantially columnar member made of a permanent magnet. The first magnetization part 21 is brazed, bonded, or welded to the main body part 25. The second magnetization part 22 is provided, in the distal end part of the main body part 25, at a position closer to the proximal end side than the first magnetization part 21 and separated from the first magnetization part 21. Similarly to the first magnetization part 21, the second magnetization part 22 is a substantially cylindrical or substantially columnar member made of a permanent magnet, and is brazed, bonded, or welded to the main body part 25. At least one of the first magnetization part 21 or the second magnetization part 22 may be formed by magnetizing a part of the main body part 25. The distal tip 24 is a member made of resin with flexibility, provided at the distal end of the main body part 25.

Here, since the guide wire 20 has a very small outer diameter while having an elongated outer shape of about several meters long, it is known that rotation at the proximal end part is not directly transmitted to the distal end part, in other words, loss torque is generated. An occurrence of the loss torque means that rotation R1 of the distal end part of the guide wire 20 is smaller than rotation R2 applied to the proximal end part of the guide wire 20 by the operator. In the medical system 1 of the present embodiment, the correspondence relation between the rotation R2 of the proximal end part of the guide wire 20 and the rotation R1 of the distal end part of the guide wire 20 following the rotation R2 is derived. Then, the medical system 1 can estimate the request operation to the above-described target operation in consideration of the loss torque by using the correspondence relation. In the present embodiment, the guide wire 20 is used as an example of the medical device, but any device such as a catheter or an endoscope may be used as the medical device.

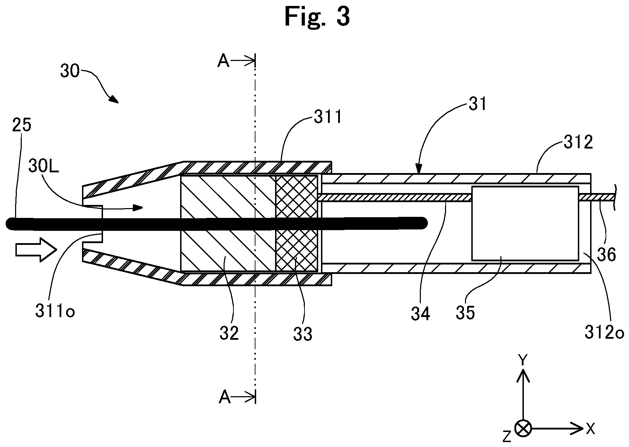

Fig. 3 is an explanatory diagram exemplifying a configuration of the holder 30. Fig. 3 illustrates X-, Y-, and Z-axes that are orthogonal to one another. The X-axis corresponds to a longitudinal direction of the holder 30, the Y-axis corresponds to a height direction of the holder 30, and the Z-axis corresponds to a width direction of the holder 30. The left side (-X-axis direction) in Fig. 3 is referred to as a "distal end side" of the holder 30 and each component member, and the right side (+X-axis direction) in Fig. 3 is referred to as a "proximal end side" of the holder 30 and each component member. In addition, one end positioned on the distal end side of the both ends in the longitudinal direction (X-axis direction) of the holder 30 and each component member is referred to as a "distal end," and the other end positioned on the proximal end side is referred to as a "proximal end." Furthermore, the distal end and a vicinity thereof are referred to as a "distal end part," and the proximal end and a vicinity thereof are referred to as a "proximal end part." Note that the X-, Y-, and Z-axes in Fig. 2 correspond to the X-, Y-, and Z-axes in Fig. 3.

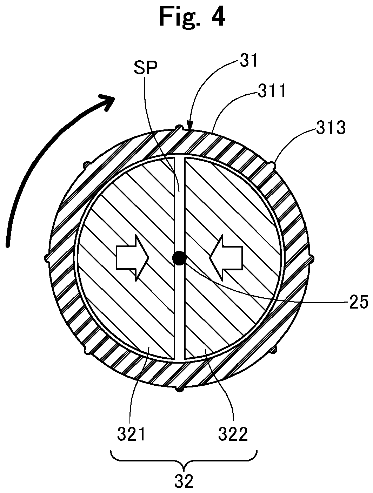

Fig. 4 is an explanatory diagram exemplifying a cross sectional configuration along a line A-A in Fig. 3. The configuration of the holder 30 will be described with reference to Figs. 3 and 4. The holder 30 is a so-called torquer that is attached to the proximal end part of the guide wire 20. As illustrated in Fig. 3, the holder 30 includes a main body part 31, a gripping part 32, a motion sensor 33, an internal connection part 34, a substrate 35, and a connection part 36.

The main body part 31 is hollow, and has an insertion path 30L for the guide wire 20 inside. The main body part 31 has a handle part 311 and a tubular part 312. The handle part 311 has a hollow and substantially cylindrical shape, in which openings are formed at a distal end and a proximal end respectively, with an inner cavity communicating both the openings with each other inside. The proximal end part of the main body part 25 of the guide wire 20 is inserted from an opening 311o at the distal end of the handle part 311 (Fig. 3: white arrow). A distal end part of the tubular part 312 is inserted into the opening at the proximal end of the handle part 311 and is brazed, bonded, or welded thereto. A part of the handle part 311 on the distal end side has a reduced diameter shape in which the outer diameter is reduced from the proximal end side to the distal end side. However, the handle part 311 may not have the reduced diameter shape, or the entire handle part 311 may have a reduced diameter shape. As illustrated in Fig. 4, a part of an outer peripheral surface of the handle part 311 has convex parts 313 that protrudes outwardly. The convex parts 313 can increase frictional force between a hand of the operator and the handle part 311, thereby improving torque transmission force from the hand of the operator to the holder 30.

As illustrated in Fig. 3, the tubular part 312 has a hollow and substantially cylindrical shape, in which openings are formed at a distal end and a proximal end respectively, with an inner cavity communicating both the openings with each other inside. The connection part 36 extends outward from an opening 312o at the proximal end of the tubular part 312 (Fig. 3). The tubular part 312 has a substantially constant outer diameter from the distal end to the proximal end, but the tubular part 312 may have a reduced diameter shape similarly to the handle part 311. The outer diameter, inner diameter, and length in the longitudinal direction of the handle part 311 and the tubular part 312 can be arbitrarily determined. The handle part 311 and the tubular part 312 may be formed as separate members and joined together as illustrated, or may be formed integrally. The handle part 311 and the tubular part 312 can be formed of any resin material. The outer peripheral surface of the handle part 311 and the convex parts 313 may be formed of rubber having elasticity, thermoplastic elastomer (TPE), or the like in order to further improve the frictional force.

The gripping part 32 is provided at a position corresponding to the inside of the handle part 311 in the insertion path 30L. As illustrated in Fig. 4, the gripping part 32 grips the proximal end part (Fig. 4: main body part 25) of the guide wire 20 inserted into the insertion path 30L. The gripping part 32 has a first gripping part 321 and a second gripping part 322 disposed with an interval SP from the first gripping part 321. The interval SP can be reduced (Fig. 4: white arrow) by rotating the handle part 311 clockwise (Fig. 4: black arrow). On the other hand, the interval SP can be increased by rotating the handle part 311 counterclockwise. In this way, the gripping part 32 grips and fixes the proximal end part of the guide wire 20.

The motion sensor 33 is attached to the gripping part 32. As is well known, the motion sensor 33 is configured by combining an acceleration sensor and a gyro sensor, and can detect a movement (acceleration, rotational angular velocity, or the like) of the proximal end part of the guide wire 20 gripped by the gripping part 32. Hereinafter, the rotational angular velocity detected by the motion sensor 33 for the rotation with the main body part 25 as a central axis (in other words, the rotation with the X-axis as a central axis) is also referred to as "proximal end rotation information". In addition, the proximal end rotation information may include the acceleration of the proximal end part of the guide wire 20 detected by the motion sensor 33 or other information that can be detected by the motion sensor 33.

The internal connection part 34 is a wiring that electrically connects the motion sensor 33 and the substrate 35. The substrate 35 is a substrate that amplifies a detection signal of the motion sensor 33 and performs analog/digital conversion (AD conversion) of the detection signal by the motion sensor 33. The substrate 35 outputs a detection value of the motion sensor 33 after the AD conversion to the computer 50 via the connection part 36. The connection part 36 is a wiring that electrically connects the substrate 35 and the computer 50. The internal connection part 34, the substrate 35, and a part of the connection part 36 are accommodated inside the tubular part 312.

Returning to Fig. 1, the description will be continued. The X-ray imaging apparatus 40 is an apparatus that acquires an X-ray image of a human body 90. The X-ray imaging apparatus 40 includes an X-ray tube apparatus, an X-ray high voltage apparatus, and an X-ray flat panel detector. The X-ray tube apparatus receives a supply of high-voltage output from the X-ray high voltage apparatus, and emits an X-ray beam. The X-ray flat panel detector converts an X-ray beam incident from the X-ray tube apparatus into an electric signal, performs analog/digital (A/D) conversion, and generates an X-ray image.

The computer 50 is an apparatus that controls the entire medical system 1, and is electrically connected to each of the magnetic sensor array 10, the holder 30, the X-ray imaging apparatus 40, the monitor 60, and the operation unit 70. The computer 50 includes a central processing unit (CPU), a read-only memory (ROM), and a random access memory (RAM), which are not illustrated. The computer 50 expands a computer program stored in the ROM to the RAM and is realized by the CPU, thereby realizing functions of a main control unit 51, a distal end information acquisition unit 52, a proximal end information acquisition unit 53, a correspondence relation deriving unit 54, a target position acquisition unit 55, and a request operation output unit 56. The main control unit 51 transmits and receives information to and from the magnetic sensor array 10, the holder 30, the X-ray imaging apparatus 40, the monitor 60, and the operation unit 70, and controls the entire medical system 1.

The distal end information acquisition unit 52 acquires information regarding a rotation movement of the distal end part of the guide wire 20. Hereinafter, the information regarding the rotation movement of the distal end part of the guide wire 20 is also referred to as "distal end rotation information". Further, the distal end information acquisition unit 52 acquires information representing a current position of the distal end part of the guide wire 20. Hereinafter, the information representing the current position of the distal end part of the guide wire 20 is also referred to as "distal end position information". The distal end information acquisition unit 52 acquires the distal end rotation information and the distal end position information by using magnetic field information acquired by a magnetic field information acquisition unit 521 and an X-ray image acquired by an X-ray image acquisition unit 522. Details will be described below. The magnetic field information acquisition unit 521 controls the magnetic sensor array 10 to acquire the magnetic field information generated by the human body 90 and the guide wire 20. The X-ray image acquisition unit 522 controls the X-ray imaging apparatus 40 to acquire the X-ray image of the human body 90.

The proximal end information acquisition unit 53 acquires information regarding the rotation movement of the proximal end part of the guide wire 20 (proximal end rotation information) from the motion sensor 33 of the holder 30.

The correspondence relation deriving unit 54 executes a derivation process described below by using the distal end rotation information acquired by the distal end information acquisition unit 52 and the proximal end rotation information acquired by the proximal end information acquisition unit 53. In the derivation process, the correspondence relation deriving unit 54 obtains a correspondence relation between the rotation movement of the proximal end part of the guide wire 20 and the rotation movement of the distal end part of the guide wire 20 following the rotation movement of the proximal end part. Details will be described below.

The target position acquisition unit 55 acquires position information of a target position of the distal end part of the guide wire 20. Here, the target position of the distal end part of the guide wire 20 means a position at which the operator intends to arrange the distal end part of the guide wire 20 in a blood vessel. In the present embodiment, an operation of moving the distal end part of the guide wire 20 from the current position to the target position is referred to as a "target operation" of the distal end part of the guide wire 20. Details will be described below.

The request operation output unit 56 obtains, by using the correspondence relation derived by the correspondence relation deriving unit 54, the position information of the target position acquired by the target position acquisition unit 55, and the distal end position information acquired by the distal end information acquisition unit 52, a request operation of the proximal end part of the guide wire 20 and outputs the obtained request operation. As described above, the "request operation" represents what kind of operation the operator should perform on the proximal end part of the medical device when the target operation is performed. Details will be described below.

The monitor 60 is a display part including a display screen 61 and is composed of a liquid crystal display or the like. The medical system 1 may include a display part other than the monitor 60. For example, the medical system 1 may include smart glasses including a display screen, or may include a projector that projects images. The operation unit 70 is composed of any means such as a keyboard, operation buttons, a touch panel, a foot switch, or a voice recognition device. The operation unit 70 is operated by the operator to switch contents displayed on the display screen 61.

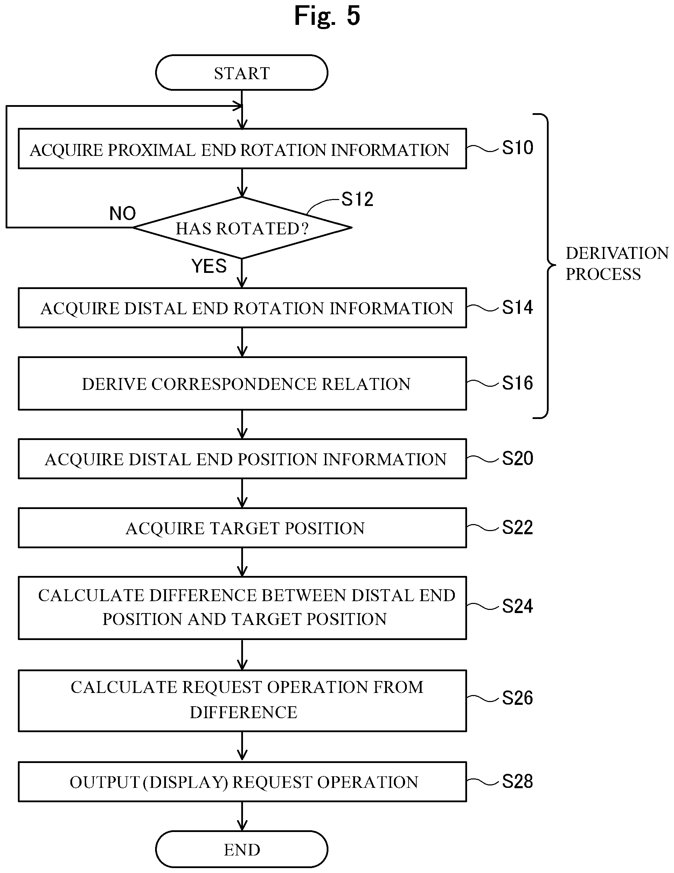

Fig. 5 is a flowchart illustrating an example of a navigation process. The navigation process is a process of outputting a request operation to the proximal end part of the guide wire 20, and is executed by the main control unit 51, the distal end information acquisition unit 52, the proximal end information acquisition unit 53, the correspondence relation deriving unit 54, the target position acquisition unit 55, and the request operation output unit 56 in cooperation with each other. The navigation process may be executed in response to an arbitrary trigger, for example, when a predetermined application installed in the computer 50 is activated.

In step S10, the proximal end information acquisition unit 53 acquires the proximal end rotation information. Specifically, the proximal end information acquisition unit 53 acquires, from the motion sensor 33 of the holder 30, the rotational angular velocity of the rotation, with the main body part 25 as a central axis, of the proximal end part of the guide wire 20, and sets the rotational angular velocity as the proximal end rotation information.

In step S12, the correspondence relation deriving unit 54 determines whether or not the proximal end part of the guide wire 20 has rotated. This determination can be performed by using the proximal end rotation information acquired in step S10. When the proximal end part has not rotated (step S12: NO), the correspondence relation deriving unit 54 shifts the process to step S10, and causes the proximal end information acquisition unit 53 to acquire the proximal end rotation information again. When the proximal end part has rotated (step S12: YES), the correspondence relation deriving unit 54 shifts the process to step S14.

In step S14, the distal end information acquisition unit 52 acquires the distal end rotation information. The distal end rotation information acquired in step S14 is caused by the rotation of the distal end part of the guide wire 20 following the rotation of the proximal end part of the guide wire 20 in step S10. Specifically, the distal end information acquisition unit 52 acquires the distal end rotation information by the following procedures a1 to a3.
(a1) The magnetic field information acquisition unit 521 of the distal end information acquisition unit 52 controls the magnetic sensor array 10 to acquire the magnetic field information from the magnetic sensor array 10. The magnetic field information includes the strength and orientation of a magnetic field obtained by combining both a biomagnetic field generated by the human body 90 and magnetic fields generated by the first magnetization part 21 and the second magnetization part 22 of the guide wire 20 inserted into the human body 90.
(a2) The X-ray image acquisition unit 522 of the distal end information acquisition unit 52 controls the X-ray imaging apparatus 40 to acquire the X-ray image from the X-ray imaging apparatus 40.
(a3) The distal end information acquisition unit 52 obtains, from the acquired magnetic field information and the X-ray image, the rotational angular velocity of the rotation, with the main body part 25 as a central axis, of the distal end part of the guide wire 20. Here, the distal end information acquisition unit 52 distinguishes and identifies a change in a magnetic field of the first magnetization part 21 and a change in a magnetic field of the second magnetization part 22 included in the magnetic field information, and thus it is possible to accurately grasp a three dimensional movement (rotation movement, forward and backward movement) of the distal end part of the guide wire 20. When the first magnetization part 21 and the second magnetization part 22 (magnetic force sources) of the guide wire 20 are permanent magnets, magnetic strength measurement values do not change due to factors (mainly time) other than a relative distance between the first magnetization part 21 and the magnetic sensor array 10 on the X-ray image and a relative distance between the second magnetization part 22 and the magnetic sensor array 10 on the X-ray image. Therefore, the distal end information acquisition unit 52 can identify the first magnetization part 21 and the second magnetization part 22 as magnetic force sources that move in conjunction with the operation of the guide wire 20 rather than a pulsation of a heart 92, in other words, magnetic force sources that move in conjunction with the movement (rotation movement, forward and backward movement) of the distal end part of the guide wire 20.

In step S16, the correspondence relation deriving unit 54 derives the correspondence relation between the rotation movement of the proximal end part of the guide wire 20 and the rotation movement of the distal end part of the guide wire 20 following the rotation movement of the proximal end part. The correspondence relation deriving unit 54 may derive the correspondence relation by using, for example, any one of methods exemplified in the following methods b1 and b2.
(b 1) The correspondence relation deriving unit 54 takes the proximal end rotation information acquired in step S10 and the distal end rotation information acquired in step S14 as teacher data to learn the correspondence relation between the rotation movement of the proximal end part of the guide wire 20 and the rotation movement of the distal end part following the rotation movement of the proximal end part by using a well-known machine learning method. In this case, the correspondence relation is realized in various modes (for example, a regression line, a neural network model, and the like) according to the learning method adopted in the correspondence relation deriving unit 54.
(b2) The correspondence relation deriving unit 54 acquires a plurality of sets of the proximal end rotation information acquired in step S10 and the distal end rotation information acquired in step S14. The correspondence relation deriving unit 54 derives the correspondence relation between the rotation movement of the proximal end part of the guide wire 20 and the rotation movement of the distal end part following the rotation movement of the proximal end part from the acquired plurality of sets by an arbitrary statistical method (for example, obtaining an average of differences).

After deriving the correspondence relation, the correspondence relation deriving unit 54 stores the obtained correspondence relation in a storage unit (not illustrated). In addition, the correspondence relation deriving unit 54 may repeatedly execute the process from Steps S10 to S14 or the process from Steps S10 to S16 until a sufficient number of teacher data is obtained. Steps S10 to S16 are also referred to as a "derivation process". In the following description, a case where the correspondence relation is derived by using the method b1 will be exemplified.

In step S20, the distal end information acquisition unit 52 acquires the distal end position information. Specifically, the distal end information acquisition unit 52 specifies the current position of the distal end part of the guide wire 20 by using the magnetic field information acquired by the magnetic field information acquisition unit 521 and the X-ray image acquired by the X-ray image acquisition unit 522. Details are the same as the procedures a1 to a3 in step S14.

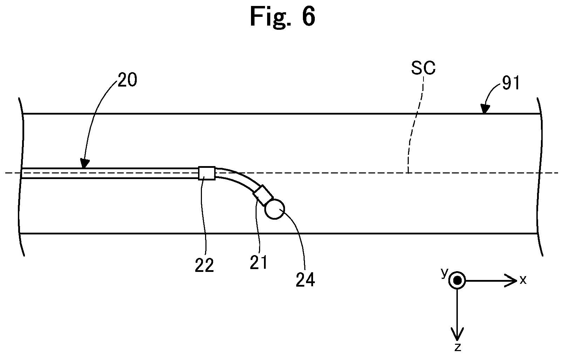

Fig. 6 is an explanatory diagram illustrating a state of the distal end part of the guide wire 20 in a blood vessel 91. As illustrated in the drawing, during use, the distal end part of the guide wire 20 may be given a small curved shape (may be pre-shaped) in order to improve blood vessel selectivity. In the example of Fig. 6, the proximal end side of the guide wire 20 is on a central axis SC of the blood vessel 91, whereas the curved distal end part is at a position deviated from the central axis SC of the blood vessel 91. Note that the x-, y-, and z-axes illustrated in Fig. 6 do not correspond to the X-, Y-, and Z-axes in Figs. 2 and 3.

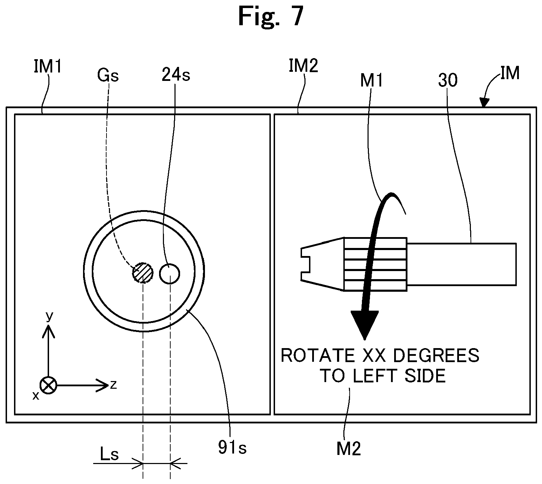

Fig. 7 is a diagram for explaining steps S22 to S28 in Fig. 5. Note that the x-, y-, and z-axes illustrated in Fig. 7 do not correspond to the X-, Y-, and Z-axes in Figs. 2 and 3. In step S22 in Fig. 5, the target position acquisition unit 55 acquires the target position. Specifically, the target position acquisition unit 55 acquires the target position by the following procedures c1 to c3. In the procedures c1 to c3 described below, the target position for positioning the distal end part of the guide wire 20 at a center of the blood vessel 91 in the case illustrated in Fig. 6 is illustrated.
(c1) The target position acquisition unit 55 acquires a contour of the blood vessel 91 in which the distal end part of the guide wire 20 is present by using the magnetic field information acquired by the magnetic field information acquisition unit 521 and the X-ray image acquired by the X-ray image acquisition unit 522.
(c2) The target position acquisition unit 55 acquires a cross section 91s (Fig. 7) of the blood vessel 91 at the position where the distal end part of the guide wire 20 is present, by using the acquired contour of the blood vessel 91 and the current position of the distal end part of the guide wire 20 based on the distal end position information acquired in step S20.
(c3) The target position acquisition unit 55 sets a center Gs of the cross section of the acquired of the blood vessel 91 as the "target position".

By changing the procedures c1 to c3, the target position acquisition unit 55 can set an arbitrary position, for example, a position where a lesion is present in the blood vessel 91 or a position where a treatment such as ablation is desired to be performed in the heart 92, as the target position.

In step S24, the request operation output unit 56 calculates a difference between a distal end position and the target position. Specifically, the request operation output unit 56 calculates the difference between a current position 24s (Fig. 7) of the distal end part of the guide wire 20 based on the distal end position information acquired in step S20 and the target position Gs (Fig. 7) of the distal end part of the guide wire 20 acquired in step S22. In the example of Fig. 7, it can be seen that the distal end position 24s is separated from the target position Gs by a distance Ls in the +z-axis direction.

In step S26, the request operation output unit 56 calculates the request operation from the difference obtained in step S24. Specifically, the request operation output unit 56 calculates the request operation by the following procedures d1 and d2.
(d1) The request operation output unit 56 obtains the target operation of the distal end part of the guide wire 20 necessary to eliminate the difference obtained in step S24 (in other words, to bring the distal end position 24s closer to the target position Gs).
(d2) The request operation output unit 56 derives an operation content (request operation) necessary for the proximal end part of the guide wire 20 in order to cause the distal end part of the guide wire 20 to perform the target operation obtained in the procedure d1 with reference to the correspondence relation derived in the derivation process. For example, in a case where the correspondence relation is realized by a neural network model (hereinafter, also referred to as an "NN model"), when a target operation of the distal end part of the guide wire 20 is input to the NN model, a request operation necessary for performing the input target operation is obtained as an output of the NN model.

In step S28, the request operation output unit 56 outputs the obtained request operation. Specifically, the request operation output unit 56 generates and outputs an image, a sound, or the like for indicating the content of the request operation to the operator. For example, the request operation output unit 56 can generate a guidance image IM illustrated in Fig. 7 and display the guidance image IM on the monitor 60. The guidance image IM in Fig. 7 includes a model image IM1 illustrating the state of the distal end part of the guide wire 20 and a model image IM2 illustrating the request operation. The model image IM1 includes an image representing the cross section 91s of the blood vessel 91 acquired in step S22, an image representing the center Gs of the cross section of the blood vessel 91 acquired in step S22, and an image representing the current position 24s of the distal end part of the guide wire 20 acquired in step S20. The model image IM2 includes an image representing the holder 30, information M1 and M2 representing the rotation direction of the proximal end part of the guide wire 20, and information M2 representing the rotation angle of the proximal end part of the guide wire 20. Note that "XX" in the information M2 means an arbitrary numerical value. The information M1 and the information M2 correspond to the "request operation".

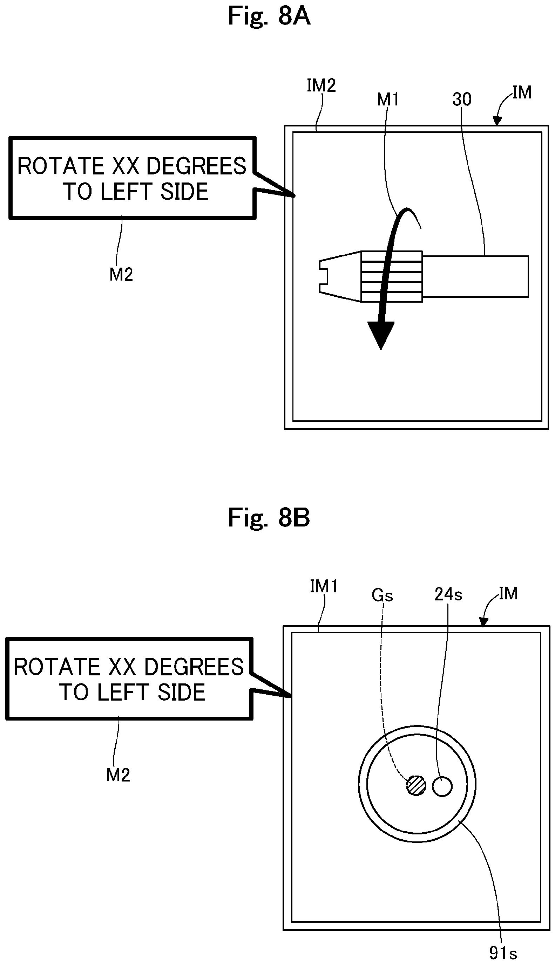

Figs. 8A and 8B illustrate another example of the image IM output to the display screen 61. Fig. 8A illustrates a first modification of the image IM, and Fig. 8B illustrates a second modification of the image IM. As illustrated in Fig. 8A, the request operation output unit 56 may display only the model image IM2 on the monitor 60. In the model image IM2, only the information M1 may be displayed on the screen, and the information M2 may be guided by other means such as voice. Further, the information M1 may be omitted. Further, as illustrated in Fig. 8B, the request operation output unit 56 may display only the model image IM1 on the monitor 60. In this case, the request operation output unit 56 guides the information M2 by other means such as voice in order to inform the operator of the request operation.

As described above, according to the medical system 1 of the first embodiment, the distal end information acquisition unit 52 can acquire quantitative distal end rotation information regarding the rotation movement of the distal end part of the guide wire 20 (medical device). Similarly, the proximal end information acquisition unit 53 can acquire quantitative proximal end rotation information regarding the rotation movement of the proximal end part of the guide wire 20. That is, according to the medical system 1, it is possible to provide the medical system 1 capable of acquiring the rotation movement of the proximal end part and the rotation movement of the distal end part of the guide wire 20. For this reason, it is possible to contribute to improvement in accuracy and improvement in efficiency of a technique by using the acquired rotation movements of the proximal end part and the distal end part in various methods. For example, the acquired rotation movements of the proximal end part and the distal end part may be directly presented to the operator. In addition, as described in the first embodiment, the acquired rotation movements of the proximal end part and the distal end part may be used to derive the rotation movement of the distal end part following the rotation movement of the proximal end part (in other words, to obtain the correspondence relation).

In addition, according to the medical system 1 of the first embodiment, the correspondence relation deriving unit 54 can derive the correspondence relation between the rotation movement of the proximal end part of the guide wire 20 and the rotation movement of the distal end part of the guide wire 20 following the rotation movement of the proximal end part by using the quantitative proximal end rotation information and distal end rotation information (Fig. 5: derivation process). Then, the request operation output unit 56 can estimate and output the "request operation of the proximal end part of the guide wire 20" in consideration of the loss torque of the guide wire 20 by using the correspondence relation derived by the correspondence relation deriving unit 54 (Fig. 7 and Figs. 8A and 8B). Here, the "request operation of the proximal end part of the guide wire 20" is a request operation of the proximal end part estimated with respect to the target operation of the distal end part of the guide wire 20, in other words, means an estimation of what kind of operation the operator performs on the proximal end part of the guide wire 20 when attempting to perform an arbitrary target operation with respect to the distal end part of the guide wire 20. As a result, according to the medical system 1 of the first embodiment, since it is possible to provide the medical system 1 capable of outputting the request operation of the proximal end part of the guide wire 20 estimated with respect to the target operation of the distal end part of the guide wire 20, it is possible to improve the accuracy of the technique and the efficiency of the technique.

In addition, the medical system 1 of the first embodiment further includes the target position acquisition unit 55 that acquires the position information of the target position of the distal end part of the guide wire 20 (medical device), and the distal end information acquisition unit 52 acquires the distal end position information representing the position of the distal end part of the guide wire 20. Therefore, the request operation output unit 56 can specify the target position Gs of the distal end part of the guide wire 20 in the cross sectional direction in the blood vessel 91 by the position information of the target position. Similarly, the request operation output unit 56 can specify the current position 24s of the distal end part of the guide wire 20 in the cross sectional direction in the blood vessel 91 by the distal end position information. As a result, the request operation output unit 56 can specify the target operation of the distal end part necessary for moving the position of the distal end part of the guide wire 20 from the current position 24s to the target position Gs, and can output the request operation necessary for the target operation.

Furthermore, according to the medical system 1 of the first embodiment, since the request operation output unit 56 outputs the rotation direction and the rotation angle of the proximal end part of the guide wire 20 (medical device) as the request operation, it is possible to clearly present a request operation content to the operator.

Further, in the medical system 1 of the first embodiment, the holder 30 attached to the proximal end part of the guide wire 20 (medical device) is configured as a so-called torquer including the hollow main body part 31, the gripping part 32 that grips the proximal end part of the guide wire 20, and the motion sensor 33 (detector) (Fig. 3). Since the proximal end information acquisition unit 53 acquires the information detected by the motion sensor 33 of the holder 30 (torquer) as the proximal end rotation information, it is possible to easily construct a function related to the proximal end information acquisition unit 53 in the medical system 1.

Furthermore, in the medical system 1 of the first embodiment, the guide wire 20 (medical device) has the first magnetization part 21 and the second magnetization part 22 that are magnetized, and the medical system 1 further includes the magnetic sensor array 10 (magnetic sensor) that detects the magnetic fields generated from the first magnetization part 21 and the second magnetization part 22. In addition, since the distal end information acquisition unit 52 calculates the distal end rotation information by using the magnetic fields detected by the magnetic sensor array 10, it is possible to easily construct a function related to the distal end information acquisition unit 52 in the medical system 1.

### <Second Embodiment>

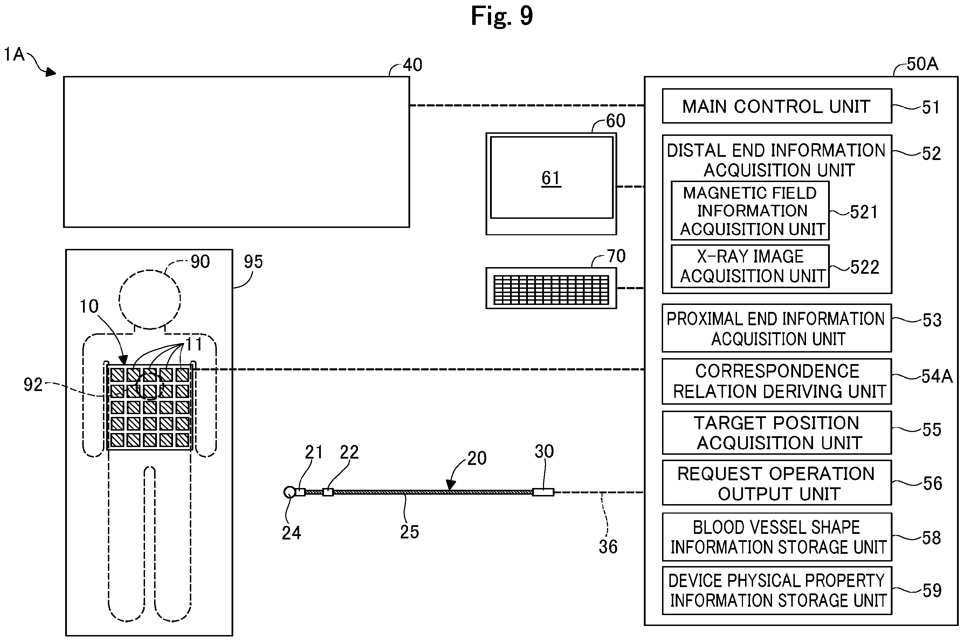

Fig. 9 is an explanatory diagram exemplifying a configuration of a medical system 1A according to a second embodiment. In the second embodiment, the accuracy of the correspondence relation by the correspondence relation deriving unit 54 can be further improved. The medical system 1A includes a computer 50A instead of the computer 50 in the configuration described in the first embodiment. The computer 50A includes a correspondence relation deriving unit 54A instead of the correspondence relation deriving unit 54, and further includes a blood vessel shape information storage unit 58 and a device physical property information storage unit 59.

The blood vessel shape information storage unit 58 stores information representing a shape of a blood vessel 91 extending at least from an insertion site for the guide wire 20 to a target site to which the distal end part of the guide wire 20 is to reach in the human body 90. The information representing the shape of the blood vessel 91 may include, for example, a curved shape of the blood vessel 91, a thickness of the blood vessel 91, and the like. The blood vessel shape information storage unit 58 may be created by image analysis of the X-ray image acquired by the X-ray imaging apparatus 40. The blood vessel shape information storage unit 58 may be created from images acquired by a computed tomography (CT) apparatus or a magnetic resonance imaging (MRI) apparatus, which are not illustrated.

The device physical property information storage unit 59 stores information representing a physical property of the guide wire 20. The information representing the physical property of the guide wire 20 may include, for example, rigidity of the guide wire 20, torque transmissibility of the guide wire 20, elasticity of the guide wire 20, and the like. In the device physical property information storage unit 59, for the guide wire 20 used in the technique, values acquired in advance by an experiment or the like or catalog values can be used.

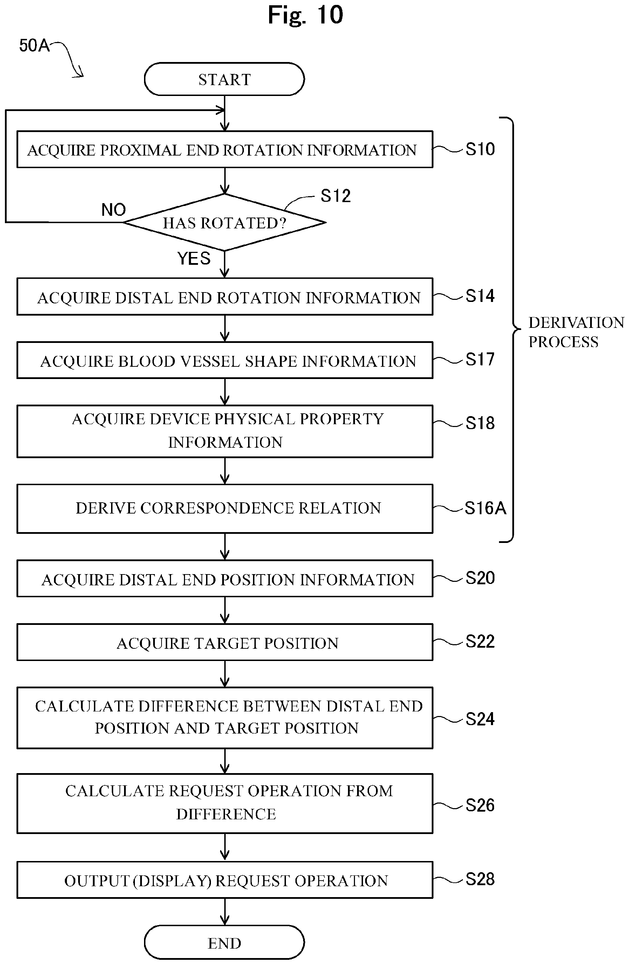

Fig. 10 is a flowchart illustrating an example of a navigation process according to the second embodiment. In Fig. 10, after the end of step S14, steps S17 and S18 are executed. In addition, step S16A is executed instead of step S16 described in the first embodiment. In step S17, the correspondence relation deriving unit 54A acquires the information (a curved shape, a thickness, or the like) regarding the shape of the blood vessel 91 in the vicinity of the distal end part of the guide wire 20 from the blood vessel shape information storage unit 58. In step S 18, the correspondence relation deriving unit 54A acquires the information (rigidity, torque transmissibility, elasticity, or the like) regarding the physical property of the guide wire 20 from the device physical property information storage unit 59. In step S16A, the correspondence relation deriving unit 54A derives the correspondence relation by using the information regarding the shape of the blood vessel 91 acquired in step S17 and the information regarding the physical property of the guide wire 20 acquired in step S18 in addition to the proximal end rotation information acquired in step S10 and the distal end rotation information acquired in step S14. In this way, the correspondence relation deriving unit 54A can derive the correspondence relation in a state in which the shape of the blood vessel 91 and the physical property of the guide wire 20 are taken into consideration.

As described above, the correspondence relation deriving unit 54A may derive the correspondence relation by adding at least one of the information regarding the shape of the blood vessel 91 or the information regarding the physical property of the guide wire 20 acquired in step S 18. Further, the correspondence relation deriving unit 54A may derive the correspondence relation by adding other information not described above. As the other information, for example, a dominant hand of the operator, a shaped shape or a shaped size of the distal end part of the guide wire 20, or the like can be used. Also, in this manner, the same effect as in the first embodiment can be obtained. Furthermore, according to the medical system 1A of the second embodiment, since the correspondence relation deriving unit 54A derives the correspondence relation by further using the information regarding the shape of the blood vessel 91 and the information regarding the physical property of the guide wire 20 (medical device), it is possible to improve the accuracy of the correspondence relation obtained by the correspondence relation deriving unit 54A. As a result, it is possible to improve estimation accuracy of the request operation to the target operation in the request operation output unit 56.

### <Third Embodiment>

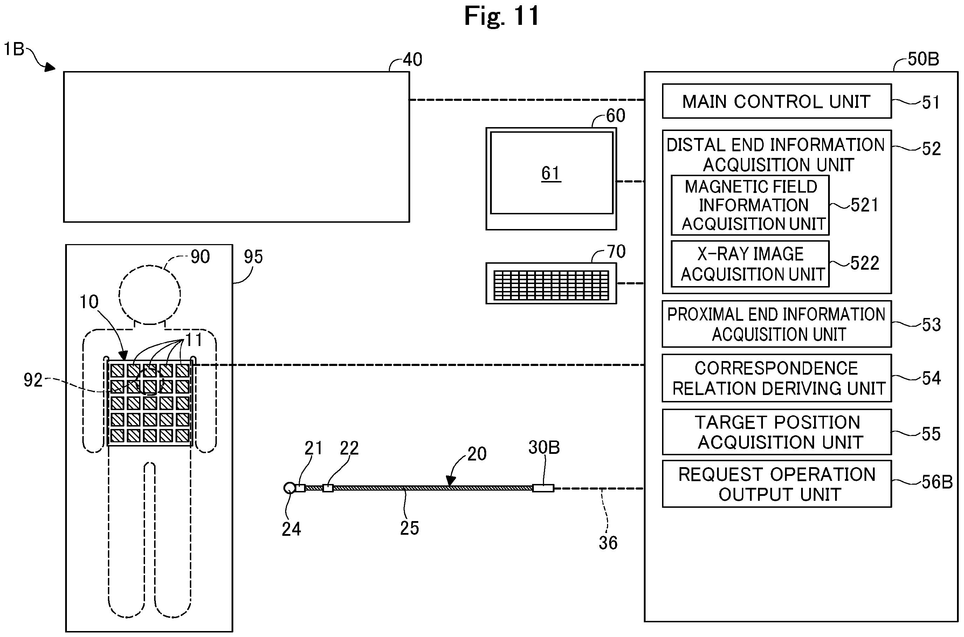

Fig. 11 is an explanatory diagram exemplifying a configuration of a medical system 1B according to a third embodiment. In the third embodiment, the guide wire 20 can be automatically operated in accordance with the request operation output by the request operation output unit 56. The medical system 1B includes a holder 30B instead of the holder 30 and a computer 50B instead of the computer 50 in the configuration described in the first embodiment. The computer 50B includes a request operation output unit 56B instead of the request operation output unit 56.

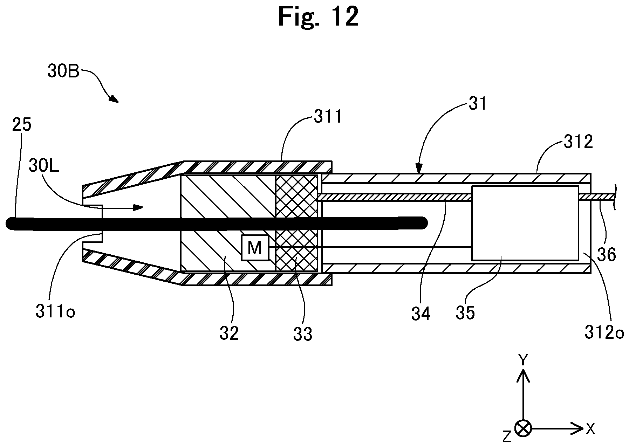

Fig. 12 is an explanatory diagram exemplifying a configuration of the holder 30B according to the third embodiment. The holder 30B further has a motor M in addition to the configuration described in the first embodiment. The motor M is connected to the gripping part 32, and rotates the gripping part 32 in the YZ-axis direction (Fig. 4: the direction of the black arrow) by being supplied with electric power from a power source which is not illustrated. When the gripping part 32 rotates, the proximal end part of the guide wire 20 gripped by the gripping part 32 also rotates. Further, the motor M is connected to the substrate 35 (control unit) that controls driving of the motor M.

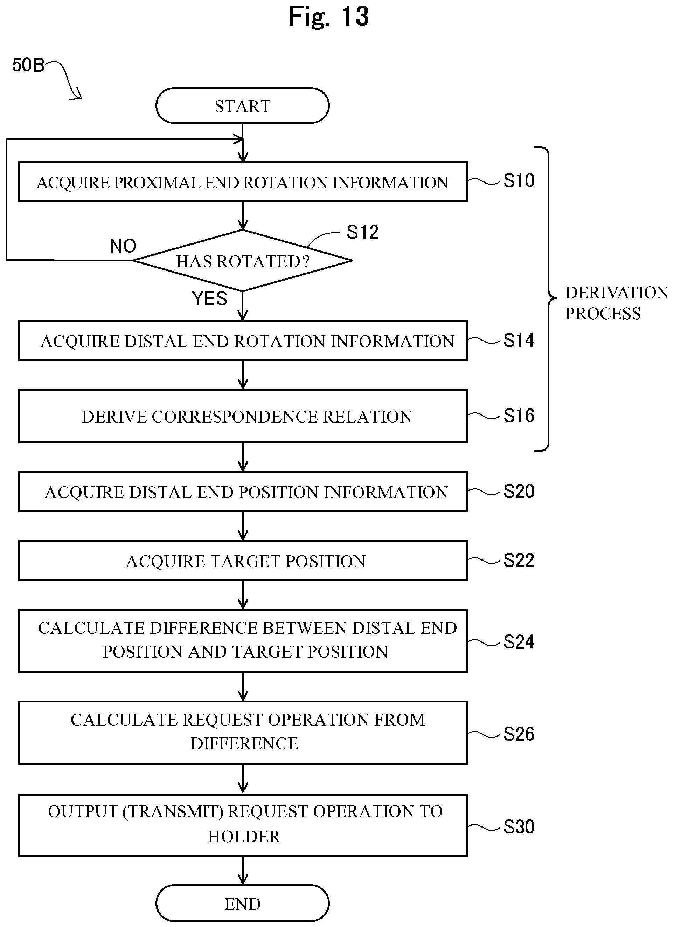

Fig. 13 is a flowchart illustrating an example of a navigation process according to the third embodiment. In Fig. 13, step S30 is executed instead of step S28 described in the first embodiment. In step S30, the request operation output unit 56B transmits the request operation obtained in step S26 to the holder 30B. The substrate 35 of the holder 30B that has received the request operation drives the motor M according to a request operation content to rotate the gripping part 32 and the guide wire 20. As a result, the proximal end part of the guide wire 20 can be operated without an operator's operation, and a delicate rotation operation can be realized as compared with a manual operation.

As described above, the motor M may be built in the holder 30B, and the request operation output unit 56B may transmit the request operation to the holder 30B. Further, in the third embodiment, an automatic operation (a configuration that drives the motor M) and a manual operation (a configuration that does not drive the motor M) may be switchable according to a preference of the operator. Also, in this manner, the same effect as in the first embodiment can be obtained. Furthermore, according to the medical system 1B of the third embodiment, the holder 30B further has the motor M that rotates the proximal end part of the gripped guide wire 20 (medical device) and the substrate 35 (control unit), and the substrate 35 controls the driving of the motor M in accordance with the request operation received from the request operation output unit 56B. Therefore, it is possible to provide the medical system 1B capable of automatically operating the proximal end part of the guide wire 20 in accordance with the request operation.

### <Fourth Embodiment>

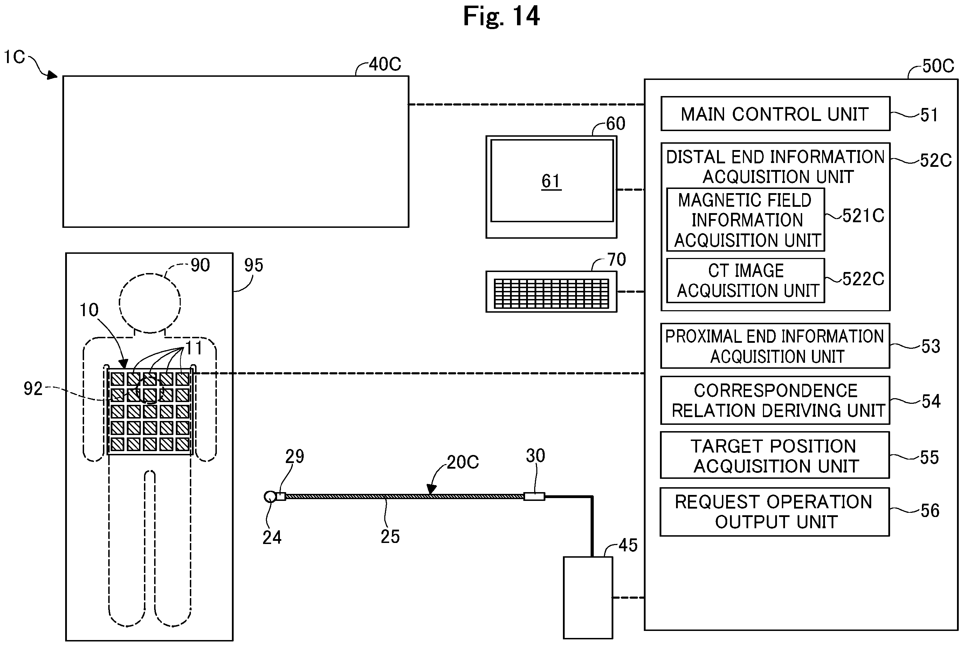

Fig. 14 is an explanatory diagram exemplifying a configuration of a medical system 1C according to a fourth embodiment. In the fourth embodiment, the same process as in the first embodiment can be performed with a configuration different from that of the first embodiment. The medical system 1C includes, in the configuration described in the first embodiment, a guide wire 20C instead of the guide wire 20, a CT apparatus 40C instead of the X-ray imaging apparatus 40, and a computer 50C instead of the computer 50.

The guide wire 20C includes, in the configuration described in the first embodiment, a conductor 29 instead of the first magnetization part 21 and the second magnetization part 22. The conductor 29 is electrically connected to a high-frequency generator 45 via the holder 30. The high-frequency generator 45 is an apparatus that supplies a high-frequency current to the guide wire 20C. The high-frequency generator 45 generates a magnetic field from the conductor 29 by supplying a current for position detection to the conductor 29. Note that the guide wire 20C may be configured to be able to ablate a biological tissue by receiving supply of a high-frequency current from the high-frequency generator 45. In this case, the distal tip 24 may be formed of a conductive material and electrically connected to the high-frequency generator 45, and a conducting return electrode may be provided outside (or a distal end part of a catheter accommodating the guide wire 20C). The high-frequency generator 45 also supplies a high-frequency current to the conducting return electrode.

The CT apparatus 40C includes, inside a gantry (mount), a tube bulb that emits X-rays and an arc-shaped detector that detects X-rays. The CT apparatus 40C generates a CT image representing the shape of the human body 90 and the shape of the heart 92 when the tube bulb rotates by 360 degrees around the human body 90 lying on the bed 95, and outputs the CT image to the computer 50.

The computer 50C has a distal end information acquisition unit 52C instead of the distal end information acquisition unit 52 in the configuration described in the first embodiment. The distal end information acquisition unit 52C has a magnetic field information acquisition unit 521C and a CT image acquisition unit 522C. The magnetic field information acquisition unit 521C acquires first magnetic field information and second magnetic field information, respectively, as the magnetic field information. Here, the "first magnetic field information" includes the strength and orientation of the biomagnetic field generated by the human body 90. The magnetic field information acquisition unit 521C acquires a detection value of the magnetic sensor array 10 in a state where no current is supplied to the conductor 29 (in other words, in a state where no magnetic field is generated from the conductor 29), and sets the detection value as first magnetic field information. The "second magnetic field information" includes the strength and orientation of a magnetic field obtained by combining both the biomagnetic field generated by the human body 90 and the magnetic field generated by the conductor 29 of the guide wire 20C inserted into the human body 90. The magnetic field information acquisition unit 521C acquires a detection value of the magnetic sensor array 10 in a state where a current is supplied to the conductor 29 (in other words, in a state where a magnetic field is generated from the conductor 29), and sets the detection value as second magnetic field information.

The distal end information acquisition unit 52C acquires the distal end rotation information by the following procedures e1 to e3 instead of the procedures a1 to a3 described in the first embodiment.
(e1) The magnetic field information acquisition unit 521C of the distal end information acquisition unit 52C controls the magnetic sensor array 10 to acquire the first magnetic field information and the second magnetic field information. The magnetic field information acquisition unit 521C compares the first magnetic field information with the second magnetic field information to acquire the strength and orientation of the magnetic field generated by the conductor 29 of the guide wire 20C. Hereinafter, it is also called "device magnetic field information".
(e2) The CT image acquisition unit 522C of the distal end information acquisition unit 52C controls the CT apparatus 40C to obtain the CT image from the CT apparatus 40C.
(e3) The distal end information acquisition unit 52C obtains, from the acquired device magnetic field information and CT image, the rotational angular velocity of the rotation, with the main body part 25 as a central axis, of the distal end part of the guide wire 20.

As described above, the distal end information acquisition unit 52C may acquire the distal end rotation information by a method different from that of the first embodiment. The conductor 29 of the guide wire 20 and the CT apparatus 40C described in the fourth embodiment are merely examples, and the distal end information acquisition unit 52C can acquire the distal end rotation information of the guide wire 20C by using various known means. For example, the distal end information acquisition unit 52C may acquire the distal end rotation information by using an MRI image acquired by an MRI apparatus instead of the CT image. Also, in this manner, the same effect as in the first embodiment can be obtained.

### <Fifth Embodiment>

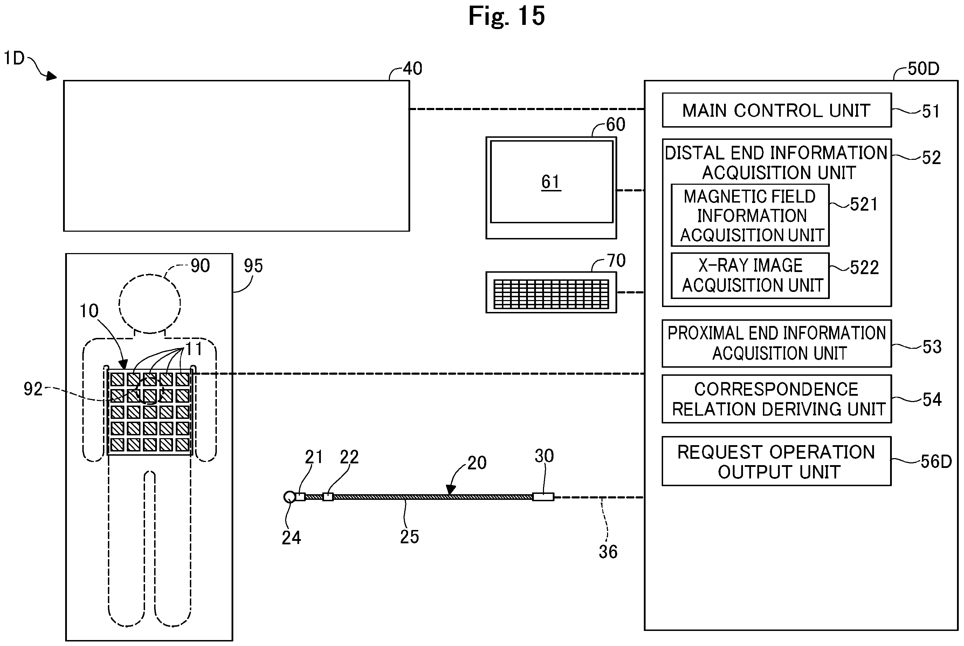

Fig. 15 is an explanatory diagram exemplifying a configuration of a medical system 1D according to a fifth embodiment. In the fifth embodiment, the request operation of the proximal end part of the guide wire 20 can be obtained without determining the target position of the distal end part of the guide wire 20. The medical system 1D includes a computer 50D instead of the computer 50 in the configuration described in the first embodiment. The computer 50D does not include the target position acquisition unit 55, and includes a request operation output unit 56D instead of the request operation output unit 56.

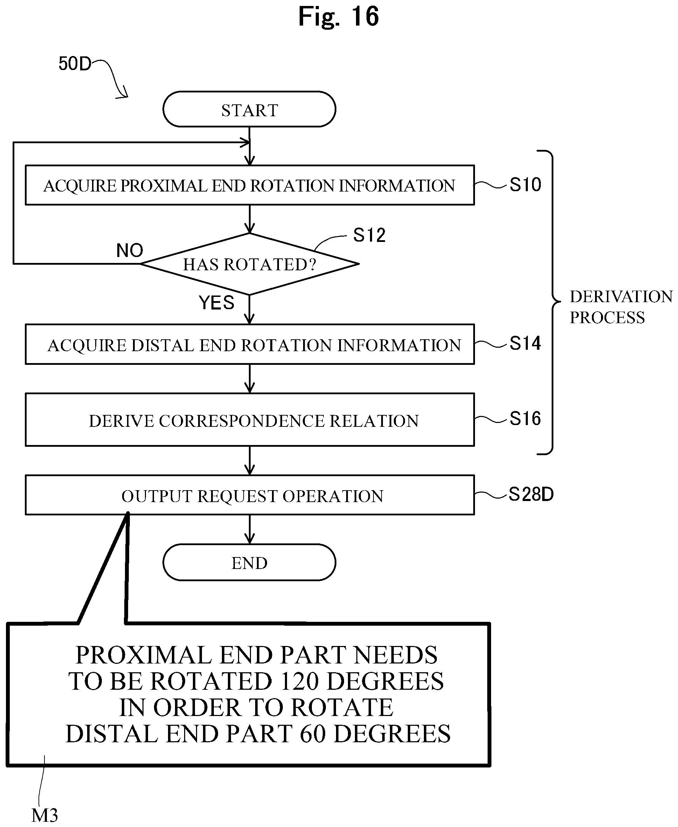

Fig. 16 is a flowchart illustrating an example of a navigation process according to the fifth embodiment. In Fig. 16, steps S20 to S26 are not executed, and step S28D is executed instead of step S28. In step S28D, the request operation output unit 56D obtains the request operation of the proximal end part of the guide wire 20 estimated to be necessary for causing the distal end part of the guide wire 20 to perform a predetermined target operation, by using the correspondence relation derived in the derivation process. The predetermined target operation may be determined in advance or may be designated by the operator. In the illustrated example, "an operation of rotating the distal end part of the guide wire 20 by 60 degrees" is exemplified as the predetermined target operation. The request operation output unit 56D outputs an obtained request operation M3 by voice. Note that the request operation output unit 56D may display the obtained request operation on the monitor 60 or on a display unit incorporated in another device such as the holder 30.

As described above, the request operation output unit 56D may obtain the request operation of the proximal end part of the guide wire 20 according to the predetermined target operation without determining the target position of the distal end part of the guide wire 20. Also, in this manner, the same effect as in the first embodiment can be obtained. Further, according to the medical system 1D of the fifth embodiment, processing contents in the computer 50D can be simplified, and processing load of the computer 50D can be reduced.

### <Sixth Embodiment>

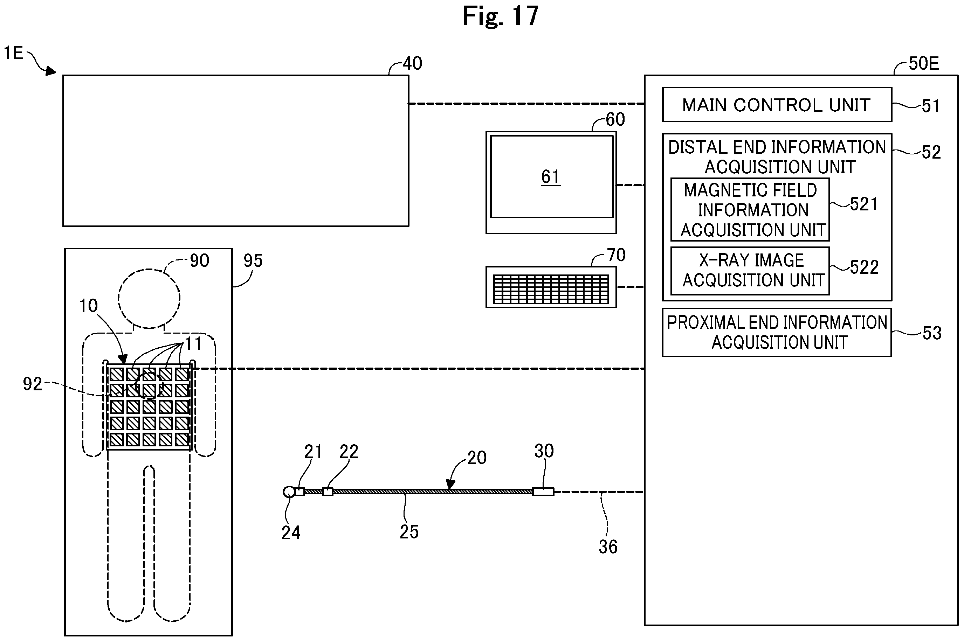

Fig. 17 is an explanatory diagram exemplifying a configuration of a medical system 1E according to a sixth embodiment. In the sixth embodiment, the derivation process is not performed, and the rotation movements of the proximal end part and the distal end part of the guide wire 20 are presented to the operator unchanged. The medical system 1E includes a computer 50E instead of the computer 50 in the configuration described in the first embodiment. The computer 50E does not include the correspondence relation deriving unit 54, the target position acquisition unit 55, and the request operation output unit 56 described in the first embodiment.

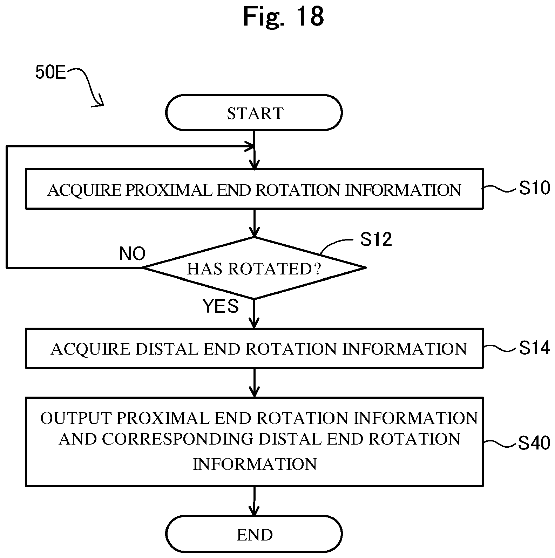

Fig. 18 is a flowchart illustrating an example of a navigation process according to a sixth embodiment. In Fig. 18, steps S16 to S26 are not executed, and step S40 is executed instead of step S28. In step S40, the main control unit 51 outputs a set of the proximal end rotation information acquired in step S10 and the distal end rotation information acquired in step S14. The output may be displayed on the monitor 60 or may be output by voice. The operator can grasp how much the distal end side rotates by how much the proximal end side of the guide wire 20 is rotated from the set of the output proximal end rotation information and the distal end rotation information. As a result, it is possible to contribute to improvement in accuracy and improvement in efficiency of the technique.

As described above, the medical system 1E may not include the correspondence relation deriving unit 54, the target position acquisition unit 55, and the request operation output unit 56 described in the first embodiment, and the processes (steps S16 to S26) realized by these functional units may be omitted. Even in this case, it is possible to provide the medical system 1E capable of acquiring the rotation movement of the proximal end part and the rotation movement of the distal end part of the guide wire 20 (medical device), so that the acquired rotation movements of the proximal end part and the distal end part can be used in various methods to contribute to improvement in accuracy and improvement in efficiency of the technique.

### <Modification of Present Embodiment>

In the embodiments described above, a part of a configuration to be achieved by a hardware may be replaced with a software, or conversely, a part of a configuration to be achieved by a software may be replaced with a hardware. Furthermore, the present inventions are not limited to the embodiments described above and may be carried out in various aspects without departing from the spirit thereof, and for example, the following modifications are also possible.

### [Modification 1]

In the first to sixth embodiments, the configurations of the medical systems 1, 1A to 1E have been exemplified. However, the configuration of the medical system 1 can be variously modified. For example, in the medical system 1, at least a part of the magnetic sensor array 10, the X-ray imaging apparatus 40, the computer 50, the monitor 60, and the operation unit 70 may be configured as an integrated device. For example, at least some of the functional units (the main control unit 51, the distal end information acquisition unit 52, the proximal end information acquisition unit 53, the correspondence relation deriving unit 54, the target position acquisition unit 55, and the request operation output unit 56) included in the computer 50 may be provided in another apparatus (a PC, a medical device, or the like) connected via a network. In this case, the computer 50 can execute the navigation process in cooperation with another device by transmitting and receiving information to and from the other device via the network.

For example, the distal end information acquisition unit 52 may acquire information regarding the rotation movement of the distal end part of the guide wire 20 (distal end rotation information) by a means different from the above-described means. Similarly, the proximal end information acquisition unit 53 may acquire the information regarding the rotation movement of the proximal end part of the guide wire 20 (proximal end rotation information) by a means different from the above-described means. Specifically, for example, at least one of the distal end rotation information or the proximal end rotation information may be input by the operator through the operation unit 70, or may be transmitted from another apparatus (a PC, a medical device, or the like) connected via a network. For example, the correspondence relation deriving unit 54 may derive the correspondence relation by means different from the above-described means b 1 and b2. Specifically, for example, a relational database in which the proximal end rotation information acquired in step S10 and the distal end rotation information acquired in step S14 are associated with each other may be created, and this database may be used as the correspondence relation.

For example, at least one of the first magnetization part 21 or the second magnetization part 22 of the guide wire 20 may be omitted. For example, the guide wire 20 may include a third magnetization part or a fourth magnetization part provided separately from the first magnetization part 21 and the second magnetization part 22. For example, the medical system 1 may be configured by using a catheter instead of the guide wire 20 described above. In this case, a magnetization part or a conductor is provided at a distal end part of the catheter, and the holder 30 is attached to a proximal end part of the catheter, whereby the same effects as in the first to sixth embodiments can be obtained. A medical device other than a catheter, such as an endoscope, may also be used.

### [Modification 2]

In the first to sixth embodiments, an example of the navigation process has been described. However, the navigation process can be variously modified, an order of steps to be executed may be changed, some steps may be omitted, and other steps which are not described may be executed. For example, an execution order of step S20 and step S22 may be reversed. For example, a date on which the derivation process of steps S10 to S16 and a date on which step S20 and subsequent steps are executed may be different. In this case, since the processes of step S20 and subsequent steps may be executed in a state in which the correspondence relation has been derived in advance, time can be shortened.

### [Modification 3]

The configurations of the medical systems 1, 1A to 1E of the first to sixth embodiments and the configurations of the modifications 1 and 2 may be appropriately combined. For example, in the medical system 1 described in the third to fifth embodiments, the derivation method described in the second embodiment (learning in consideration of the blood vessel shape information storage unit 58 and the device physical property information storage unit 59) may be adopted. For example, in the configurations described in the fourth and fifth embodiments, the automatic operation of the proximal end part of the guide wire 20 described in the third embodiment may be adopted. For example, in the configurations described in the fifth and sixth embodiments, the method of detecting the distal end rotation information described in the fourth embodiment may be adopted.

Although the aspects have been described based on the embodiments and the modifications, the embodiments of the above-described aspects are for facilitating understanding of the aspects, and do not limit the aspects. The aspects can be modified and improved without departing from the spirit of the aspects and the scope of claims, and include equivalent aspects. Furthermore, the technical features may be omitted as appropriate unless they are described as essential in this specification.

### DESCRIPTION OF REFERENCE NUMERALS

1, 1A to 1E Medical system
10 Magnetic sensor array
11 Magnetic sensor
20, 20C Guide wire
21 First magnetization part
22 Second magnetization part
24 Distal tip
25 Main body part
29 Conductor
30, 30B Holder
31 Main body part
32 Gripping part
33 Motion sensor
34 Internal connection part
35 Substrate
36 Connection part
40 X-ray imaging apparatus
40C CT apparatus
45 High-frequency generator
50, 50A to 50D Computer
51 Main control unit
52, 52C Distal end information acquisition unit
53 Proximal end information acquisition unit
54, 54A Correspondence relation deriving unit
55 Target position acquisition unit
56, 56B, 56D Request operation output unit
58 Blood vessel shape information storage unit
59 Device physical property information storage unit
60 Monitor
61 Display screen
70 Operation unit
90 Human body
91 Blood vessel
92 heart
95 bed
311 Handle part
312 tubular part
313 Convex part
321 First gripping part
322 Second gripping part
521, 521C Magnetic field information acquisition unit
522 X-ray image acquisition unit
522C CT image acquisition unit

## Claims

1. A medical system comprising:
a distal end information acquisition unit that acquires distal end rotation information regarding a rotation movement of a distal end part of a medical device to be inserted into a living body; and
a proximal end information acquisition unit that acquires proximal end rotation information regarding a rotation movement of a proximal end part of the medical device to be operated by an operator.

2. The medical system according to claim 1, further comprising:
a correspondence relation deriving unit that derives, by using the proximal end rotation information and the distal end rotation information, a correspondence relation between the rotation movement of the proximal end part of the medical device and the rotation movement of the distal end part of the medical device following the rotation movement of the proximal end part; and
a request operation output unit that outputs, by using the correspondence relation derived by the correspondence relation deriving unit, a request operation of the proximal end part of the medical device, which is estimated with respect to a target operation of the distal end part of the medical device.

3. The medical system according to claim 2, wherein
the request operation output unit outputs, as the request operation, an operation of the proximal end part of the medical device, which is estimated with respect to the target operation of the distal end part necessary for moving a position of the distal end part in a cross sectional direction in a blood vessel from a current position to a target position by the rotation movement of the distal end part of the medical device,
the medical system further comprises a target position acquisition unit that acquires position information of the target position,
the distal end information acquisition unit further acquires distal end position information representing a current position of the distal end part, and
the request operation output unit outputs the request operation by using the position information of the target position and the distal end position information.

4. The medical system according to claim 2 or 3, wherein
the request operation output unit outputs, as the request operation, a rotation direction and a rotation angle of the proximal end part of the medical device.

5. The medical system according to any one of claims 2 to 4, wherein
the correspondence relation deriving unit further derives the correspondence relation by using information regarding a shape of the blood vessel and information regarding a physical property of the medical device.

6. The medical system according to any one of claims 1 to 5, further comprising:
the medical device with a holder attached to the proximal end,
wherein the holder has:
a hollow main body part that has an insertion path for the medical device;
a gripping part that is provided in the insertion path and grips the proximal end part of the medical device; and
a detector that detects information regarding the rotation movement of the proximal end part of the medical device gripped by the gripping part,
the proximal end information acquisition unit acquires information detected by the detector as the proximal end rotation information.

7. The medical system according to claim 6, wherein
the holder further has:
a motor that rotates the proximal end part of the medical device gripped by the gripping part; and
a control unit that controls driving of the motor,
the request operation output unit transmits the request operation to the control unit, and
the control unit controls the driving of the motor in accordance with the request operation received.

8. The medical system according to any one of claims 1 to 7, further comprising:
a magnetic sensor,
wherein the medical device has:
a first magnetization part that is provided at the distal end part and magnetized; and
a second magnetization part that is provided closer to a proximal end side than the first magnetization part and magnetized,
the magnetic sensor detects magnetic fields generated from the first magnetization part and the second magnetization part, and
the distal end information acquisition unit calculates the distal end rotation information by using the magnetic fields detected by the magnetic sensor.

9. A navigation method comprising:
acquiring distal end rotation information regarding a rotation movement of a distal end part of a medical device to be inserted into a living body; and
acquiring proximal end rotation information regarding a rotation movement of a proximal end part of the medical device to be operated by an operator.
